# EUROPEAN PATENT SPECIFICATION

(11) **EP 4 069 192 B1**
(45) Date of publication and mention of the grant of the patent: **16.09.2026**
(21) Application number: 20820564.1
(22) Date of filing: 02.12.2020
(51) Int. Cl.: A61K 9/00, A61K 31/00, A61K 9/68, A61K 9/02, A61K 9/20

(54) **(S)-3-[1-METHYLPYRROLIDIN-2-YL]PYRIDINE, ANALOGUES THEREOF, PRECURSORS THEREOF, OR ITS DERIVATIVES, FOR THE USE AS A PHARMACEUTICAL IN FORM OF A SOLID SUBSTANCE AND A TABLET**
(S)-3-[1-METHYLPYRROLIDIN-2-YL]PYRIDIN, ANALOGA DAVON, VORLÄUFER DAVON ODER SEINE DERIVATE ZUR VERWENDUNG ALS ARZNEIMITTEL IN FORM EINER FESTEN SUBSTANZ UND EINER TABLETTE
(S)-3-[1-MÉTHYLPYRROLIDIN-2-YL]PYRIDINE, ANALOGUES CORRESPONDANTS, PRÉCURSEURS CORRESPONDANTS, OU SES DÉRIVÉS, POUR L'UTILISATION EN TANT QUE PRODUIT PHARMACEUTIQUE SOUS LA FORME D'UNE SUBSTANCE SOLIDE ET D'UN COMPRIMÉ

(30) Priority: 02.12.2019 LU 101519
(43) Date of publication of application: 12.10.2022
(73) Proprietor: Solis Herrera, Arturo, CP20000 Aguascalientes (MX)
(72) Inventor: Solis Herrera, Arturo, CP20000 Aguascalientes (MX)
(74) Representative: Bals & Vogel Patentanwälte PartGmbB
(86) International application number: PCT/IB2020/061402
(87) International publication number: WO 2021/111340

(56) References cited:
- WO-A2-2004/056363
- US-A- 5 662 920
- US-A1- 2014 088 044
- US-A1- 2018 303 818
- FLOOD PAMELA ET AL: "Intranasal nicotine for postoperative pain treatment", ANESTHESIOLOGY, AMERICAN SOCIETY OF ANESTHESIOLOGISTS, US, vol. 101, no. 6, 1 December 2004 (2004-12-01), pages 1417 - 1421, XP009116941, ISSN: 0003-3022, DOI: 10.1097/00000542-200412000-00023
- RICHARD A GLENNON: "Nicotine and Pain", MEDICINAL CHEMISTRY RESEARCH, BIRKHÄUSER-VERLAG, BOSTON, vol. 13, no. 1-2, 1 January 2004 (2004-01-01), pages 74 - 77, XP019202293, ISSN: 1554-8120, DOI: 10.1007/S00044-004-0011-0

## Description

The present invention relates to (S)-3-[1-Methylpyrrolidin-2-yl]pyridine pharmaceutical. In addition, a tablet is disclosed, but not claimed.

### Field of invention

(S)-3-[1-Methylpyrrolidin-2-yl]pyridine, analogues thereof, precursors thereof, or its derivatives, also known as nicotine, is an alkaloid found predominantly in plants like tobacco and cocoa and in lower quantities even in plants like tomato, potato, eggplant and green pepper, all belonging to the nightshade family of plants (Solanaceae). (S)-3-[1-Methylpyrrolidin-2-yl]pyridine is accumulated in the leaves of these plants while its biosynthesis is taken place in the particular roots. Thereby, (S)-3-[1-Methylpyrrolidin-2-yl]pyridine constitutes approximately 0,6 to 0,3% of the dry weight of for example the tobacco plant and is present in the range of 2 to 7 µg/KG of various edible plants.

Further, (S)-3-[1-Methylpyrrolidin-2-yl]pyridine is a stimulant drug acting as an agonist to most nicotinic acetylcholine receptors (nAChR). In lesser doses of approximately 1 mg the substance adds on as a stimulant in mammals, while high amounts of approximately 50 to 100 mg can start being harmful.

(S)-3-[1-Methylpyrrolidin-2-yl]pyridine is hygroscopic, oily, colorless or pale yellow liquid, which is miscible with water and in its base form comprises a pyridine odor. (S)-3-[1-Methylpyrrolidin-2-yl]pyridine comprises a molecular weight of approximately 172 g/mol, is readily soluble in alcohol, ether or light petroleum. Further, (S)-3-[1-Methylpyrrolidin-2-yl]pyridine forms salts with acids that are solid and water soluble. The substance is further absorbed from the gastro-intestinal tract, and also by the respiratory tract and the mucosa. Thereby, (S)-3-[1-Methylpyrrolidin-2-yl]pyridine undergoes extensive first pass metabolism when administered orally. Furthermore, (S)-3-[1-Methylpyrrolidin-2-yl]pyridine easily penetrates the skin, particularly the mucosa. The substance is able of being quickly distributed throughout the bloodstream and is even able to cross the blood-brain-barrier. The half-life of (S)-3-[1-Methylpyrrolidin-2-yl]pyridine in the body is approximately two hours.

(S)-3-[1-Methylpyrrolidin-2-yl]pyridine mentioned within the scope of the invention is always understood as (S)-3-[1-Methylpyrrolidin-2-yl]pyridine itself, analogues thereof, precursors thereof, its derivatives or nicotine which mimic the effect of (S)-3-[1-Methylpyrrolidin-2-yl]pyridine, either alone or in combination with other active substances can be used.

### Background of the invention

In US 5,549,906 A the use of nicotine lozenges is provided as a method of reducing craving for nicotine and of smoking cessation therapy. Therewith a method for producing transient blood levels of nicotine mimicking the effects of cigarette smoking is allocated, comprising the periodic use of nicotine lozenges to provide rapid delivery of nicotine to the buccal mucosa. The nicotine lozenges are designed to be held in the patient's mouth and sucked, and to release nicotine into the buccal cavity. The disadvantage of such a delivery system is that nicotine is used for smoking cessation therapy, wherein the use of nicotine is only administered when the craving for a cigarette is increased.

In "Intranasal Nicotine for Postoperative Pain Treatment", DOI: 10.1097/00000542-200412000-00023, the potential to use an intranasal nicotine spray for its analgesic effect using a single 3 mg dose administered to women before their emergence from general anesthesia post-surgery is disclosed. It is found that treating female patients with a single dose of nicotine prior to the emergence from anesthesia lead to a significant reduction in pain scores during the first day post-surgery while not increasing the risks of hypertension of tachycardia.

In "Nicotine and Pain", DOI: 10.1007/S00044-004-0011-0, the history of the use of nicotine as an antinociceptive compound is disclosed.

In US2018303818A1, compositions and methods that are composed of nicotinic agonists capable of treating nervous system disorders are disclosed, in particular combination therapies.

US5662920A discloses a method for smoking cessation that utilizes an improved nicotine-based lozenge containing nicotine, sweetener and an absorbent.

In WO2004056363A2, a nicotine-containing particulate material for the release of nicotine is disclosed, where the material comprises a combination of nicotine or its derivatives and a microcrystalline cellulose. The particulate material is stable and releases nicotine relatively fast.

In US2014088044A1, a product comprising a nicotine-containing material and an anti-cancer agent is disclosed. The product is applicable in the treatment and/or prevention of cancer and precancerous conditions as well as for preventing cancer recurrence.

### Detailed description of the invention

This problem is solved according to the invention by all features of claim 1.

The special benefit of this invention is that a certain dosage of (S)-3-[1-Methylpyrrolidin-2-yl]pyridine, analogues thereof, precursors thereof or its derivatives, is administered as defined by the claims and can be reliably delivered to the desired destination within the body.

Advantageously, a solid substance can contain divers concentrations of (S)-3-[1-Methylpyrrolidin-2-yl]pyridine, analogues thereof, precursors thereof or its derivatives, in order to be absorbed by the body. For oral preparations, which are disclosed but not claimed, (S)-3-[1-Methylpyrrolidin-2-yl]pyridine can be used alone or in combination with appropriate additives to make tablets, powders, granules or capsules, for example, with conventional additives, such as lactose, mannitol, corn starch or potato starch, with binders, such as crystalline cellulose, cellulose derivatives, acacia, corn starch or gelatins, with disintegrators, such as corn starch, potato starch or sodium carboxymethylcellulose, with lubricants, such as talc or magnesium stearate, and if desired, with diluents, buffering agents, moistening agents, preservatives and flavoring agents. The solid substance thereby comprises discrete particles of (S)-3-[1-Methylpyrrolidin-2-yl]pyridine. Generally, solid substances vary in their disparity and comprise a whole range of particle sizes. Advantageously, these particles are of a size, which can be processed as powder or a tablet, so that the substance can be delivered to the body, particularly the digestive tract and/or be absorbed by the mucosa.

In one embodiment not covered by the claimed invention (S)-3-[1-Methylpyrrolidin-2-yl]pyridine is intended as a tablet. Further, in one possible embodiment not covered by the invention (S)-3-[1-Methylpyrrolidin-2-yl]pyridine, analogues thereof, precursors thereof or its derivatives, is intended that the substance is a powder. Alternatively and/or additionally, (S)-3-[1-Methylpyrrolidin-2-yl]pyridine may be in form of a salt, which is solid at room temperature. Therefore, (S)-3-[1-Methylpyrrolidin-2-yl]pyridine like its salt formulations are generally soluble in water. Beneath (S)-3-[1-Methylpyrrolidin-2-yl]pyridine also pharmacological analogues or derivatives or substances which mimic the effect of (S)-3-[1-Methylpyrrolidin-2-yl]pyridine, either alone or in combination with other active substances can be used. In general, tablets are suspended within the mouth of a human and/or animal. A tablet is a form of a pharmaceutical dosage. In general tablets can be produced in any shape. However, due to requirements of patients and tableting machines most are round, oval or in a capsuled shaped. Advantageously tablets are easy to swallow. Further the diameter and the shape of the tablets are determined by the producing machines. The process of making a tablet by powder compaction is very common. First, the powder is filled into the desired from above. The amount of powder is determined by the to be produced tablet. Preferably it has to be ensured that fluctuations in tablet weight, usually caused by uneven powder flow have to be avoided. Further also fluctuations in dosage of the active pharmaceutical ingredient need to be prevented. Advantageously the tablets are coated. Thereby the coating is of a sugar and/or a polymer and/or a polysaccharide base. Preferably plasticizers and pigments can be included. Thereby the coatings are stable and strong enough to survive the handling of the tablet. Further a sticking together of tables during is prevented. Thereby coatings are necessary for tablets that have an unpleasant taste. Further a coating makes tablets easier to swallow. Tablet coatings are further an advantage to enhance the stability of the tablet and protect against moisture or oxidation. If the active ingredient of a tablet is sensitive to acid, or is irritant to the stomach lining, an enteric coating can be used, which is resistant to stomach acid, and dissolves in the less acidic area of the intestines. Thereby coatings can be chosen in order to control the rate of dissolution of the drug in the gastrointestinal tract. Some drugs will be absorbed better at different points in the digestive system. If the highest percentage of absorption of a drug takes place in the stomach, a coating that dissolves quickly and easily in acid will be selected. If the rate of absorption is best in the large intestine or colon, then a coating that is acid resistant and dissolves slowly would be used to ensure it reached that point before dispersing.

In general solid substances are applied to the oral cavity of an individual. Hereby, the substance containing particles of (S)-3-[1-Methylpyrrolidin-2-yl]pyridine is administered to the oral cavity of the individual so that it is capable of being absorbed. Therefore, the particles are of an adequate size for absorption. Thereby the aerosol can be in form of a powder or a tablet, which contains the substance in a solid manner, particularly a carrier. Said adequate size of particles regards to solid components within the substance. However, it is provided that the sizes are valid for the migration through the oral cavity into the digestive tract. A preferred size is thereby approx. 1µm to approx. 15µm in diameter. Preferably, the size is between approx. 5µm to approx. 10µm in diameter.

Advantageously, in an embodiment that is disclosed but not claimed, (S)-3-[1-Methylpyrrolidin-2-yl]pyridine, analogues thereof, precursors thereof or its derivatives, is intended for a suppository. A suppository is a drug delivery system that is inserted into the body, particularly in the rectum, the vagina or the urethra, where it dissolves or melts and is absorbed into the blood stream. Suppositories can be used to deliver both systemically and locally acting medications. Preferably a suppository is made from a greasy base, like cocoa butter, or from a water soluble base, such as polyethylene glycol, or from glycerol and gelatin, which can melt at body temperature. Advantageously suppositories are easier to administer than tablets or syrups, particularly when an individual has a vomiting tendency. Moreover some active substances are even better tolerated in suppository form. The application of the substances as a suppository allows an uptake into the bloodstream where the substance can affect the organism and thereby reduce the symptoms of diverse diseases. It is an advantage of an application with a suppository that no digestion is required and the effective substance can be applied in the vicinity of the target tissue. The substance is directly transferred via the bloodstream to the predetermined pharmacological target location. Further, a reliable intake occurs.

Furthermore, (S)-3-[1-Methylpyrrolidin-2-yl]pyridine, analogues thereof, precursors thereof or its derivatives, in an embodiment that is disclosed but not claimed, is intended for retarded release. A retarded release provides that the active substance is not absorbed at once, but delivered at least two points of a time. Advantageously a retarded release ensures that that a single intake of the active substance leads to an abortion for a period of time.

(S)-3-[1-Methylpyrrolidin-2-yl]pyridine, analogues thereof, precursors thereof or its derivatives, in an embodiment that is disclosed but not claimed, is intended for a substance which contains a vehicle substance, particularly starch and/or amtodextrin. Generally excipients can include diluents, binders or granulating agents, glidants and lubricants to ensure efficient use as a powder or a tablet. Thereby disintegrants can be used to promote tablet break-up in the digestive tract. Moreover also sweeteners or flavors can be used to enhance the taste and have a pleasant perception while intaking the active substance. Further pigments can be used in order to make the tablets visually attractive and distinguishable. A polymer coating is often applied to make the tablet smoother and easier to swallow, to control the release rate of the active ingredient and to make it more resistant to the environment. Further, common minerals like talc or silica, and fats, e.g. vegetable stearin, magnesium stearate or stearic acid are the most frequently used lubricants in tablets or hard gelatin capsules.

In one embodiment not covered by the claimed invention (S)-3-[1-Methylpyrrolidin-2-yl]pyridine, analogues thereof, precursors thereof or its derivatives comprise a concentration of 0,3 mg/ml to 4,5 mg/ml, preferably 1,5 mg/ml to 3,0 mg/ml. Herein, the delivery system applies a dose of the active agent of about 0,3 mg/ml to 4,5 mg/ml per unit dose. Preferably, 1,5 mg/ml to 3,0 mg/ml is applied. Advantageously, the applied concentration of (S)-3-[1-Methylpyrrolidin-2-yl]pyridine is not toxic. Using a cytotoxic compound can result in a variety of cell fates. The appearance of cytotoxic concentrations can result in necrosis (loss of membrane integrity) and thereby cell lysis, stopping cell growth and dividing or even apoptosis. In order to investigate the cytotoxicity level of certain substances cytotoxicity assays are widely used in the pharmaceutical industry. Further indicators being investigated in order to determine the level of cytotoxicity are for example TNFα (tumor necrosis factor alpha), IFNγ (Interferon gamma) or IL-6 (interleukin). TNFα is a cell signaling protein mainly involved in systemic inflammation. Cells that produce TNFα are mainly macrophages, lymphocytes, mast cells, or neurons. However, the main function of TNFα is in the regulation of immune cells. Further, IFNγ is a soluble cytokine. IFNγ is a cytokine that is critical for immunity against viral, bacterial and protozoal infections. Thereby it is an important activator of macrophages and inducer of MHC (major histocompatibility complex) expression. IFNγ has antiviral, immunoregulatory and anti-tumor properties. Further IL-6 is an interleukin that acts as both a pro-inflammatory cytokine and an anti-inflammatory myokine. In mammalian IL-6 is secreted by T-cells and macrophages to stimulate an immune response.

(S)-3-[1-Methylpyrrolidin-2-yl]pyridine, analogues thereof, precursors thereof or its derivatives, triggers stimulation of cholinergic receptors, particularly nicotinic receptors. Nicotine acetylcholine receptors (nAChR) are neuronal receptor proteins that signal upon a chemical stimulus. The cholinergic receptors are forming ligand gated ion channels in the plasma membrane of the certain neuron. They are expressed on the presynaptic and postsynaptic side of the neuro muscular junctions. As neurotropic receptors they are directly linked to ion channels and thereby are not dependent on second messengers. Neuron receptors are found in the central nervous system as well as in the peripheral nervous system of mammalians. Generally, ligand gated ion channels require the binding of a chemical messenger for opening. The endogenous agonist is acetylcholine wherein they are also triggered by (S)-3-[1-Methylpyrrolidin-2-yl]pyridine, analogues thereof, precursors thereof or its derivatives. The action that (S)-3-[1-Methylpyrrolidin-2-yl]pyridine binds to nicotine cholinergic receptors can be regarded as a key energy source of the cell, as this is responsible for upregulating of energy. All nicotine cholinergic receptors produce excitatory postsynaptic effects. Thereby, energy is upregulated previous to an excitatory postsynaptic effect. Further, the administration of (S)-3-[1-Methylpyrrolidin-2-yl]pyridine upregulates the release of endogenous alpha-MSH. The melanocytes stimulating hormone (MSH) belong to peptide hormones that are produced in the central nervous system. These hormones stimulate the production inducing melanin by melanocytes in the skin and in the hair. Alpha-MSH induces multiple responses in nearly all cells of mammalians, thereby significantly improving and also maintaining good health conditions. Alpha-MSH is further related to chemical energy levels within the mammalian body and thereby fundamental for an optimal body performance.

In one advantageous embodiment, which is disclosed but not claimed, of the invention is (S)-3-[1-Methylpyrrolidin-2-yl]pyridine. analogues thereof, precursors thereof or its derivatives is used for the treatment of diseases related to pain. Further, these substances also modulate many disease pathologies like viral related diseases which are H1N1 intoxications, which are according to the claims. Further disclosed but not claimed disease pathologies relate to skin physiology, melanocytes function, nerve regeneration, behavior and learning as well as memory, obesity and energy metabolism, brain inflammation, autoimmune diseases, septic shock, endotoxemia, renal ischemic occlusion and reperfusion, mesenteric occlusion and reperfusion, diabetes, viral related intoxications like Ebola, congenital mal formations and cancer as well as inflammatory diseases. Particularly, the substance is disclosed but not claimed to be related to diseases as joint pain and further to diseases related to nerves as well as to skin. Mainly, these diseases can be treated in mammalians preferably in humans and/or animals.

(S)-3-[1-Methylpyrrolidin-2-yl]pyridine, analogues thereof, precursors thereof or its derivatives, are administered every 24 hours. Preferably, the substance is administered every 12 hours, preferably every 6 hours. Thereby, the frequency of administration depends upon the variety of disease. In administration every 2 to 3 hours is likewise possible.

The present disclosure not covered by the scope of the claimed invention further relates to a tablet with a substrate, which suspends within the mouth of a human and/or animal. A tablet is a form of a pharmaceutical dosage. Thereby tablets include pills, capsules and variants thereof like caplets (smooth, coated, oval-shaped medicinal tablet). Generally, tablets comprise an active substance as well as excipients. A tablet further is usually generated in form of a powder, which is pressed or compacted into a solid dose. A tablet is formulated to deliver an accurate dosage to a specific site. Usually tablets are taken orally, but can also be administered sublingually, buccally, rectally or intravaginally. Generally a tablet is a medication that can be digested. Advantageously tablets rapidly dissolves in saliva or are not dissolved until they are digested. The sizes of the tablet ranges from a 1 mm to approx. 1 cm. Further, the tablet delivers a measured dosage of a pharmacological relevant substances. The tablet is thereby suitable for applying (S)-3-[1-Methylpyrrolidin-2-yl]pyridine, analogues thereof, precursors thereof or its derivatives. Advantageously, tablets are simple and convenient to use.

It is an advantage when the substance is dispensed in a single dosage. Each tablet provides a distinct dosage of the active ingredient, preferably in a convenient portable package. Further, they can be chewable as a tablet or as a gum. Chewable devices base on an ionexchange resin that releases nicotine slowly when the patient chews, and the nicotine present in the mouth is delivered directly to the systemic circulation by buccal absorption and/or is swallowed and delivered to the intestinal tract. Further, tablets can be designed to protect unstable medications or disguise unpalatable ingredients. Moreover, tablets can be configured with colored coatings or embossed markings or a printing in order to aid tablet discrimination and recognition. Therefor a tablet can preferably be wrapped in a blister package.

The present invention is further described in detail with respect to the accompanying examples and figures. The features mentioned in the claims and specifications are essential to the invention, either in themselves or in given combination.

### Examples and Figures

In a first example according to the scope of the claimed invention, a treatment with (S)-3-[1-Methylpyrrolidin-2-yl]pyridine of viral diseases like for example H1N1 was tested on chicken, infected with the H1N1 virus. Therefore, 30000 diseased chicken were divided into two groups of 15000. One group was treated with (S)-3-[1-Methylpyrrolidin-2-yl]pyridine at doses of 0,002 mg/kg each 24 hours. The other group represented the control group. 24 hours after treatment the treated group began to lay off eggs in contrary to the control group, which turned down the production of eggs. As comparative example, the treatment against intoxication was tested related to mercury, CN, cadmium, arsenic, herbicides, pesticides. As a comparative example mercury poisoning induces a generalized failure, typical to low levels of energy, so that the mortality level is high. As a result 6 in 8 rats died at the dose of 4,5 mg/kg of mercury (HgHCI). In any of the organs edema and hemorrhage was examined. After a treatment of 0,0937 mg/ml with (S)-3-[1-Methylpyrrolidin-2-yl]pyridine mortality was reduced to 0,625 of 8 rats.

### Figures

- Fig. 1: a diagram showing a treatment of a group of humans (n = 10/each) ulcer with (S)-3-[1-Methylpyrrolidin-2-yl]pyridine
- Fig. 2a: a diagram showing a cytotoxicity test with WI-38 cells treated with (S)-3-[1-Methylpyrrolidin-2-yl]pyridine
- Fig. 2b: a diagram showing a cytotoxicity test with A549 cells treated with (S)-3-[1-Methylpyrrolidin-2-yl]pyridine
- Fig. 2c: a diagram showing a cytotoxicity test with HS683 cells treated with (S)-3-[1-Methylpyrrolidin-2-yl]pyridine
- Fig. 3: a diagram showing a cytotoxicity test of human PBLs treated with (S)-3-[1-Methylpyrrolidin-2-yl]pyridine
- Fig. 4a: a diagram showing the influence of (S)-3-[1-Methylpyrrolidin-2-yl]pyridine on the proliferation of WI-38 cells
- Fig. 4b: a diagram showing the influence of (S)-3-[1-Methylpyrrolidin-2-yl]pyridine on the proliferation of A549 cells
- Fig. 4c: a diagram showing the influence of (S)-3-[1-Methylpyrrolidin-2-yl]pyridine on the proliferation of HS683 cells
- Fig. 5a: a diagram showing the effect of (S)-3-[1-Methylpyrrolidin-2-yl]pyridine on TNF-α production
- Fig. 5b: a diagram showing the effect of (S)-3-[1-Methylpyrrolidin-2-yl]pyridine on IFN-γ production

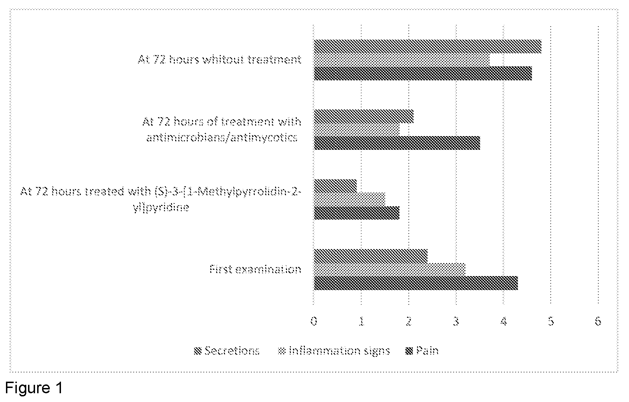

In a further comparative example (Fig. 1) the effect of (S)-3-[1-Methylpyrrolidin-2-yl]pyridine for use in treating the healing process of an ulcer is examined as a basic condition. Different ulcers (n = 10 for each group) are examined each after 72 h treatment with (S)-3-[1-Methylpyrrolidin-2-yl]pyridine (Group A), treatment with antimicrobians/antimycotics (group B) and without treatment (group C). As a result the treatment with (S)-3-[1-Methylpyrrolidin-2-yl]pyridine resulted in an increased healing effect (group A) compared to the treatment with antimicrobians/antimycotics (group B) and without treatment (group C) (Fig. 1).

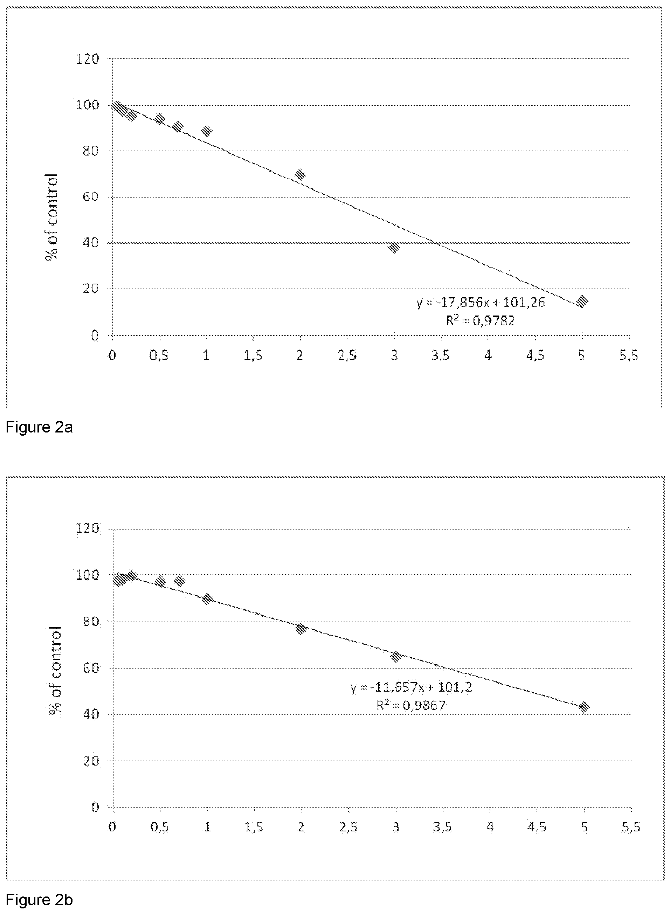

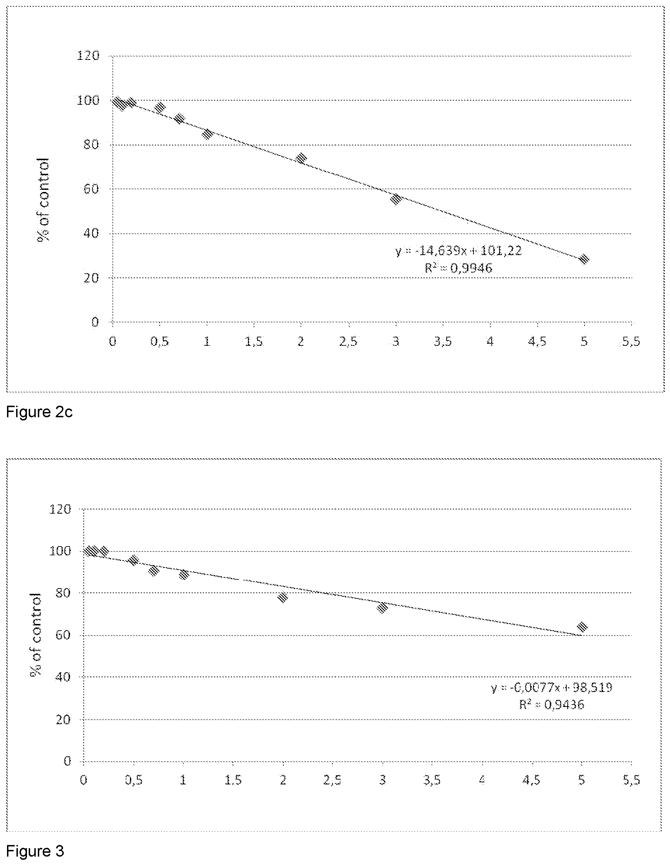

In a further comparative example cytotoxicity tests were performed with three different cell lines (Fig. 2a, 2b, 2c):
- WI-38 (human diploid cell line from normal embryonic lung tissue)
- A549 (human lung adenocarcinoma epithelial cell line) and
- HS683 (human neuronal glioma cell line).

Thereby, cell viability was determined on the basis of the mitochondria-dependent reduction of MTT (3-[4,5-dimethyltiazol 2yl]-2,5-diphenyltetrazolium bromide) to formazan. Cell lines (2x10⁵cell/ml) as well as leukocytes (2x10⁶ cells/ml) were cultured in 96-well plates for 48 hours in the presence of various concentrations of (S)-3-[1-Methylpyrrolidin-2-yl]pyridine (5-0,05 mg/ml). Next cells were treated with 20 µl of MTT. After 3 hours of incubation at 37°C, the formazan blue formed in the cells was dissolved in 100 µl of SDS (sodium dodecylsulfate) for 3 hours in 37°C. The optical density was measured at 570 nm.

After the cytotoxicity tests LC₅₀ (LC₅₀-lethal concentration, 50%-dose required to kill half of the cells after a specified test duration) were estimated for each cell lines and PBLs (leukocytes of peripheral blood). Additionally for PBLs LC₁₀ and LC₅ were calculated. On the basis of the results for leukocytes three different concentrations of (S)-3-[1-Methylpyrrolidin-2-yl]pyridine were selected for evaluation of the effect of cytokine production.

Further, a proliferation test was prepared for the same three cell lines: WI-38, A549 and HS 683. 3x10⁴ WI-38 cells and 2x10⁴ A549 and HS 683. Cells were seeded into each wells in 96-well culture plates in appropriate culture medium with 10% FBS (fetal bovine serum). After 24 h incubation in 37°C/5%CO₂ medium were removed and replaced with tested concentrations of (S)-3-[1-Methylpyrrolidin-2-yl]pyridine in culture medium. After 96 hours incubation in 37°C/5%CO₂ MTT assay was performed for establishing the cell viability.

Studies of the effect of (S)-3-[1-Methylpyrrolidin-2-yl]pyridine on cytokine production was performed on PBLs isolated ex vivo from 10 healthy volunteers (male 5; female 5; age 23-45). Next, leukocytes were treated with selected, nontoxic concentrations of (S)-3-[1-Methylpyrrolidin-2-yl]pyridine (1 mg/ml; 500 µg/ml, 300 µg/ml) without or with 5 µg/ml of LPS (lipopolysaccharide). After 24 hours of incubation in 37°C/5%CO₂ samples of leukocytes and supernatants were collected. For evaluation of the influence of (S)-3-[1-Methylpyrrolidin-2-yl]pyridine on IFN-γ (interferon) production additionally samples were collected after 72 hours of incubation.

Cytokine levels were determined using ELISA kits (BD Biosciences, USA) for human IFNγ, TNF-α and IL-6 and DuoSet for human IL-3 (R&D Systems, USA) according to the producers' instructions. The optical density was measured at 450 nm using a Multiskan RC spectrophotometric reader (Thermo Labsystems, USA), Cytokine concentrations were expressed in pg/ml.

In summary the LC₅₀ of (S)-3-[1-Methylpyrrolidin-2-yl]pyridine for a cell line WI-38 is 3,0 mg/ml, for the cell line A549 is 4,5 mg/ml and for the cell line HS-683 is 3,5 mg/ml.

In Figure 3 the results of the cytotoxicity test of human PBLs is shown.

On the basis of the results of MTT assay for human leukocytes the LC₅₀, LC₁₀ and LC₅ were evaluated. The LC₅₀ is a concentration of 7 mg/ml (S)-3-[1-Methylpyrrolidin-2-yl]pyridine. The LC₁₀ is in a concentration of 1 mg/ml and LC₅ is in a concentration of 0,5 mg/ml (S)-3-[1-Methylpyrrolidin-2-yl]pyridine.

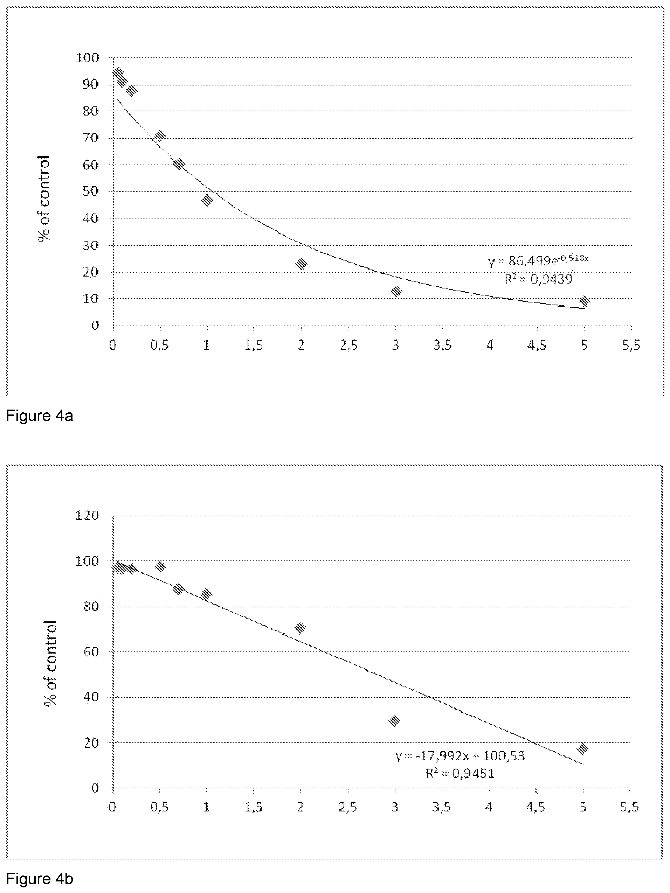

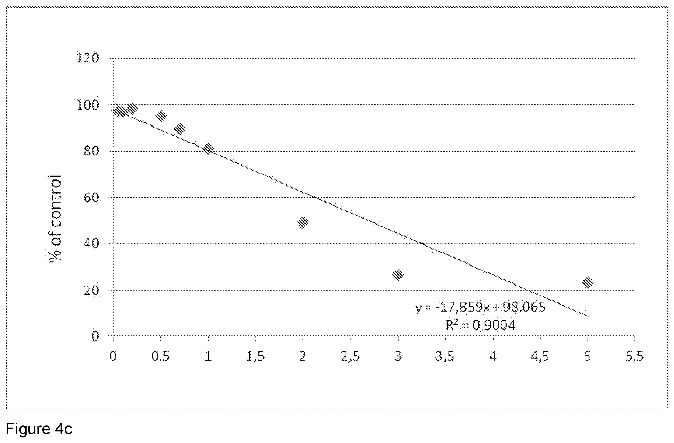

In a further comparative example (Figures 4a to 4c) the influence of (S)-3-[1-Methylpyrrolidin-2-yl]pyridine on the proliferation of divers cell lines was investigated. Figure 3a shows the effect on WI-38 cells. For the cell line WI-38 1,0 mg/ml of (S)-3-[1-Methylpyrrolidin-2-yl]pyridine inhibits 50% of the proliferation of these cells, whereby 4,5 mg/ml inhibits a proliferation of 99%.

For the cell line A549 a concentration of 2,0 mg/ml (S)-3-[1-Methylpyrrolidin-2-yl]pyridine inhibits 50% of the proliferation whereas the concentration of 5,5 mg/ml inhibits 99% of the proliferation.

For the cell line HS-683 2,9 mg/ml (S)-3-[1-Methylpyrrolidin-2-yl]pyridine inhibits 50% of the proliferation of these cells wherein 99% of proliferation are inhibited in a concentration of 5,5 mg/ml.

In another comparative example the cytokine production is evaluated utilizing the effect of (S)-3-[1-Methylpyrrolidin-2-yl]pyridine on TNF-α production. As shown in Figure 4a the preparation increases the level of cytokines in a dose-dependent manner. The effect was observed only for unstimulated human PBLs.

At the same time studies of the effect of (S)-3-[1-Methylpyrrolidin-2-yl]pyridine on IFN-γ production showed that the preparation strongly decreases the level of interferon in a dose-dependent manner. The effect was observed for LPS stimulated human PBLs. There were poor or no spontaneous IFN-γ production after 24 hours and 72 hours of incubation of PBLs. At the same time no effect of (S)-3-[1-Methylpyrrolidin-2-yl]pyridine was observed for IL-6 production by human PBLs.

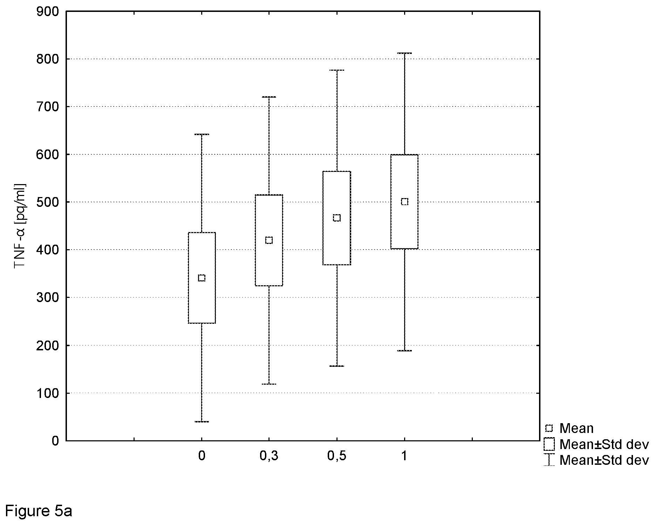

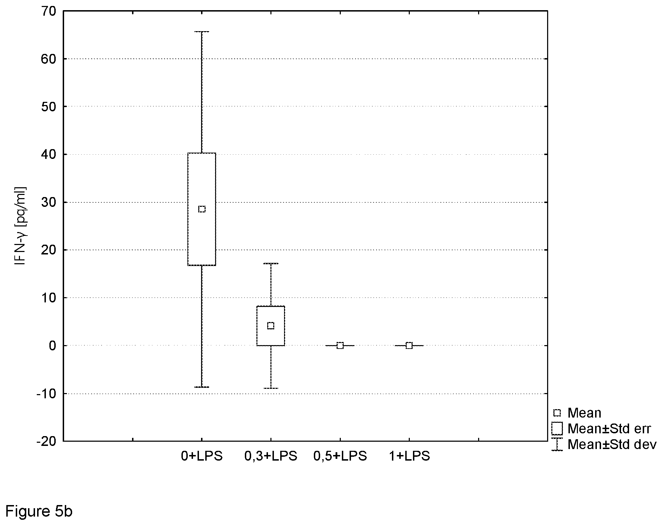

Shown in Figures 1 to 5 allow cytotoxicity of (S)-3-[1-Methylpyrrolidin-2-yl]pyridine was observed. The preparation inhibits 50% of cell proliferation in doses higher than 1 mg/ml. All other concentrations were harmless. Preparation increased TNF-α production by unstimulated human PBLs in a dose-dependent manner. Further, (S)-3-[1-Methylpyrrolidin-2-yl]pyridine strongly reduces IFN-γ production by LPS-stimulated human PBLs. The effect was again dose-dependent.

## Claims

1. (S)-3-[1-Methylpyrrolidin-2-yl]pyridine as a pharmaceutical for the use in treatment of H1N1 viral disease in chickens, wherein the concentration of (S)-3-[1-Methylpyrrolidin-2-yl]pyridine administered is 0.002 mg/kg each 24 hours.

## Patentansprüche

1. (S)-3-[1-Methylpyrrolidin-2-yl]pyridin als pharmazeutisches Mittel zur Behandlung von H1N1-Viruserkrankung bei Hühnern, wobei die verabreichte Konzentration von (S)-3-[1-Methylpyrrolidin-2-yl]pyridin 0,002 mg/kg jede 24 Stunden beträgt.

## Revendications

1. (S)-3-[1-méthylpyrrolidin-2-yl]pyridine en tant que produit pharmaceutique pour le traitement d'une maladie virale H1N1 chez des poulets, la concentration de (S)-3-[1-méthylpyrrolidin-2-yl]pyridine administrée étant de 0,002 mg/kg toutes les 24 heures.
